(19) Europäisches Patentamt
European Patent Office
Office européen des brevets

(11) **EP 2 973 095 B1**

(12) **EUROPEAN PATENT SPECIFICATION**

(45) Date of publication and mention of the grant of the patent:
**09.05.2018 Bulletin 2018/19**

(51) Int Cl.:
***G06F 19/00*** (2018.01)

(21) Application number: **14716046.9**

(22) Date of filing: **28.02.2014**

(86) International application number:
**PCT/US2014/019377**

(87) International publication number:
**WO 2014/149536 (25.09.2014 Gazette 2014/39)**

(54) **INSULIN TIME-ACTION MODEL**

**ZEITAKTIONSMODELL FÜR INSULIN**

**MODÈLE TEMPS-ACTION D'INSULINE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361787658 P**

(43) Date of publication of application:
**20.01.2016 Bulletin 2016/03**

(73) Proprietor: **Animas Corporation West Chester, PA 19380 (US)**

(72) Inventor: **MORALES, Carlos West Chester, Pennsylvania 19380 (US)**

(74) Representative: **Carpmaels & Ransford LLP One Southampton Row London WC1B 5HA (GB)**

(56) References cited:
**WO-A1-03/080157** **WO-A1-2010/135646**
**US-A1- 2011 208 156**

• COBELLI C ET AL: "Diabetes: Models, Signals, and Control", IEEE REVIEWS IN BIOMEDICAL ENGINEERING, IEEE, USA, vol. 2, 1 January 2009 (2009-01-01), pages 54-96, XP011285638, ISSN: 1937-3333

• MATTHEW W PERCIVAL ET AL: "Closed-Loop Control and Advisory Mode Evaluation of an Artificial Pancreatic [beta] Cell: Use of Proportional-Integral-Derivative Equivalent Model-Based Controllers Author Affiliations: Corresponding Author", JOURNAL OF DIABETES SCIENCE AND TECHNOLOGY VOLUME DIABETES TECHNOLOGY SOCIETY, vol. 2, no. 4, 1 July 2008 (2008-07-01), pages 636-644, XP055137504,

• CHIARA DALLA ET AL: "Physical Activity into the Meal Glucose-Insulin Model of Type 1 Diabetes: In Silico Studies Author Affiliations", JOURNAL OF DIABETES SCIENCE AND TECHNOLOGY VOLUME DIABETES TECHNOLOGY SOCIETY, vol. 3, no. 1, 1 January 2009 (2009-01-01) , pages 56-67, XP055137742,

• MALGORZATA E WILINSKA ET AL: "Simulation Environment to Evaluate Closed-Loop Insulin Delivery Systems in Type 1 Diabetes Author Affiliations", JOURNAL OF DIABETES SCIENCE AND TECHNOLOGY VOLUME DIABETES TECHNOLOGY SOCIETY, vol. 4, no. 1, 1 January 2010 (2010-01-01) , XP055137833,

• Anonymous: "Bayesian network", Wikipedia, 10 March 2013 (2013-03-10), XP055351419, Retrieved from the Internet: URL:https://en.wikipedia.org/w/index.php?t itle=Bayesian_network&oldid=543217952 [retrieved on 2017-03-03]

**Description**

**BACKGROUND**

Diabetes mellitus is a chronic metabolic disorder caused by an inability of the pancreas to produce sufficient amounts of the hormone insulin, resulting in the decreased ability of the body to metabolize glucose. This failure leads to hyperglycemia, i.e. the presence of an excessive amount of glucose in the blood plasma. Persistent hyperglycemia and/or hypoinsulinemia has been associated with a variety of serious symptoms and life-threatening long-term complications such as dehydration, ketoacidosis, diabetic coma, cardiovascular diseases, chronic renal failure, retinal damage and nerve damages with the risk of amputation of extremities. Because restoration of endogenous insulin production is not yet possible, a permanent therapy is necessary which provides constant glycemic control in order to always maintain the level of blood glucose within normal limits. Such glycemic control is achieved by regularly supplying external insulin to the body of the patient to thereby reduce the elevated levels of blood glucose.

External biologic agents such as insulin have commonly been administered as multiple daily injections of a mixture of rapid- and intermediate-acting drugs via a hypodermic syringe. It has been found that the degree of glycemic control achievable in this way is suboptimal because the delivery is unlike physiological hormone production, according to which hormone enters the bloodstream at a lower rate and over a more extended period of time. Improved glycemic control may be achieved by the so-called intensive hormone therapy which is based on multiple daily injections, including one or two injections per day of a long acting hormone for providing basal hormone and additional injections of rapidly acting hormone before each meal in an amount proportional to the size of the meal. Although traditional syringes have at least partly been replaced by insulin pens, the frequent injections are nevertheless very inconvenient for the patient, particularly those who are incapable of reliably self-administering injections.

Substantial improvements in diabetes therapy have been achieved by the development of drug delivery devices that relieve the patient of the need for syringes or drug pens and the need to administer multiple daily injections. The drug delivery device allows for the delivery of a drug in a manner that bears greater similarity to the naturally occurring physiological processes and can be controlled to follow standard or individually-modified protocols to give the patient better glycemic control.

In addition, delivery directly into the intraperitoneal space or intravenously can be achieved by drug delivery devices. Drug delivery devices can be constructed as an implantable device for subcutaneous arrangement or can be constructed as an external device with an infusion set for subcutaneous infusion to the patient via the transcutaneous insertion of a catheter, cannula or a transdermal drug transport such as through a patch. External drug delivery devices are mounted on clothing, hidden beneath or inside clothing, or mounted on the body, and are generally controlled via a user interface built in to the device or arranged on a separate remote device.

Blood or interstitial glucose monitoring is required to achieve acceptable glycemic control. For example, delivery of suitable amounts of insulin by the drug delivery device requires that the patient frequently determine his or her blood glucose level and manually input this value into a user interface for the external pumps. The user interface or a corresponding controller then calculates a suitable modification to the default or currently in-use insulin delivery protocol, i.e., dosage and timing, and subsequently communicates with the drug delivery device to adjust its operation accordingly. The determination of blood glucose concentration is typically performed by means of an episodic measuring device such as a hand-held electronic meter which receives blood samples via enzyme-based test strips and calculates the blood glucose value based on the enzymatic reaction.

Continuous glucose monitoring (CGM) has also been utilized over the last twenty years with drug delivery devices to allow for closed loop control of the insulin(s) being infused into the diabetic patients. To allow for closed-loop control of the infused insulins, proportional-integral-derivative ("PID") controllers have been utilized with mathematical model of the metabolic interactions between glucose and insulin in a person. The PID controllers can be tuned based on simple rules of the metabolic models. However, when the PID controllers are tuned or configured to aggressively regulate the blood glucose levels of a subject, overshooting of the set level can occur, which is often followed by oscillations, which is highly undesirable in the context of regulation of blood glucose. Model predictive controllers ("MPC") have also been used. The MPC controller has been demonstrated to be more robust than PID because MPC considers the near future effects of control changes and constraints in determining the output of the MPC, whereas PID typically involves only past outputs in determining future changes. MPC therefore is more effective than PID in view of the complex interplay between insulin, glucagon, and blood glucose. Constraints can be implemented in the MPC controller such that MPC prevents the system from running away when a control limit has been reached. For example, some schemes do not deliver any glucose during a hypoglycemic excursion. Another benefit of MPC controllers is that the model in the MPC can, in some cases, theoretically compensate for dynamic system changes whereas a feedback control, such as PID control, such dynamic compensation would not be possible.

Additional details of the MPC controllers, variations on the MPC and mathematical models representing the complex interaction of glucose and insulin are shown and described in the following documents:

US Patent No. 7,060,059;

U.S. Patent No. 8,062,249;

US Patent Application Nos. 2011/0313680 and 2011/0257627,

International Publication WO 2012/051344, US Publication 2011/0208156;

International Publication WO 2003/080157 and WO2010/135646;

Percival et al., "Closed-Loop Control and Advisory Mode Evaluation of an Artificial Pancreatic β Cell: Use of Proportional-Integral-Derivative Equivalent Model-Based Controllers" Journal of Diabetes Science and Technology, Vol. 2, Issue 4, July 2008.

Paola Soru et al., "MPC Based Artificial Pancreas; Strategies for Individualization and Meal Compensation" Annual Reviews in Control 36, p.118-128 (2012),

Cobelli et al., "Artificial Pancreas: Past, Present, Future" Diabetes Vol. 60, Nov. 2011;

Magni et al., "Run-to-Run Tuning of Model Predictive Control for Type 1 Diabetes Subjects: In Silico Trial" Journal of Diabetes Science and Technology, Vol. 3, Issue 5, September 2009.

Lee et al., "A Closed-Loop Artificial Pancreas Using Model Predictive Control and a Sliding Meal Size Estimator" Journal of Diabetes Science and Technology, Vol. 3, Issue 5, September 2009;

Lee et al., "A Closed-Loop Artificial Pancreas based on MPC: Human Friendly Identification and Automatic Meal Disturbance Rejection" Proceedings of the 17th World Congress, The International Federation of Automatic Control, Seoul Korea July 6-11, 2008;

Magni et al., "Model Predictive Control of Type 1 Diabetes: An in Silico Trial" Journal of Diabetes Science and Technology, Vol. 1, Issue 6, November 2007;

Wang et al., "Automatic Bolus and Adaptive Basal Algorithm for the Artificial Pancreatic β-Cell" Diabetes Technology and Therapeutics, Vol. 12, No. 11, 2010;

Percival et al.., "Closed-Loop Control of an Artificial Pancreatic β-Cell Using MultiParametric Model Predictive Control" Diabetes Research 2008;

Cobelli et al., "Diabetes: Models, Signals, and Control", IEEE Reviews in Biomedical Engineering, Vol. 2, 2009;

Percival et al., "Closed-Loop Control and Advisory Mode Evaluation of an Artificial Pancreatic [beta] Cell: Use of Proportional-Integral-Derivative Equivalent Model-Based Controllers", Journal of Diabetes Science and Technology, Vol. 2, Issue. 4, July 2008;

Dalla Man et al.: "Physical Activity into the Meal Glucose-Insulin Model of Type 1 Diabetes: In Silico Studies Author Affiliations", Journal of Diabetes Science and Technology, Vol. 3, Issue 1, January 2009; and

Wilinska et al.: "Simulation Environment to Evaluate Closed-Loop Insulin Delivery Systems in Type 1 Diabetes Author Affiliations", Journal of Diabetes Science and Technology, Vol. 4, Issue 1, January 2010. Document WO03/080157 is considered as closest prior art and discloses an MPC controller, but does not disclose the exponential function as in claim 1, used for MPC control.

Drug delivery devices generally provide insulin at a "basal rate," i.e., provide a certain amount of insulin every few minutes in a pre-programmed, daily pattern. Some drug delivery devices also permit the user to specify a "temporary basal," in which the normal daily cycle is altered for a selected length of time.

Some drug delivery devices permit the user to manually request that a "bolus," a specified amount of insulin, be delivered at a specified time. For example, before a meal, the user can request a bolus of additional insulin be delivered to process the glucose produced by digestion of the meal. Some drug delivery devices permit the specified amount to be delivered over a period of time rather than all at once; time-extended delivery is referred to as an "extended bolus."

The effects of basals and boluses of insulin vary by patient and by type of insulin. Moreover, the appearance of insulin into the blood stream (pharmacokinetic: how the drug is absorbed by or distributed through the body) is different than the measurement of insulin action (pharmacodynamic: the effects of the drug once administered). Pharmacodynamics are often measured using a euglycemic clamp technique. In this technique, insulin is injected into a subject so that the subject's liver is no longer producing glucose from glycogen. Glucose is then supplied to the subject intravenously, and the rate of glucose supply is controlled to maintain the subject's blood glucose level at a normal ("euglycemic") level. The rate of glucose supply at any given time indicates how much glucose the subject's body is using at that time. Since the liver responds to insulin by converting glucose into glycogen, the rate at which glucose is supplied to hold blood glucose is correlated with the effect of the insulin at reducing blood sugar.

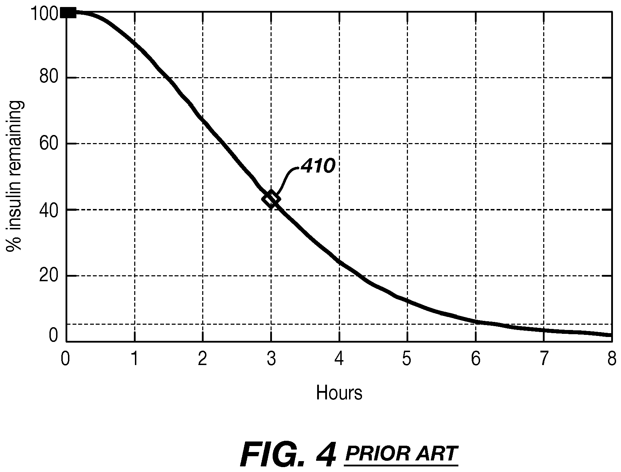

FIG. 4 shows an example of timing of insulin action for insulin aspart from a euglycemic clamp (0.2 U/kg into the abdomen). Using this graph assists patients to avoid "insulin stacking." For example, 3 hours after administration of 10 units of insulin aspart, one can estimate that there is still 40% of the 10 units, or 4 units of insulin remaining (point 410). By way of comparison, the pharmacodynamic duration of action of regular insulin is approximately twice that of insulin aspart or insulin lispro. Currently used insulin pumps keep track of this "insulin-on-board" to avoid insulin stacking.

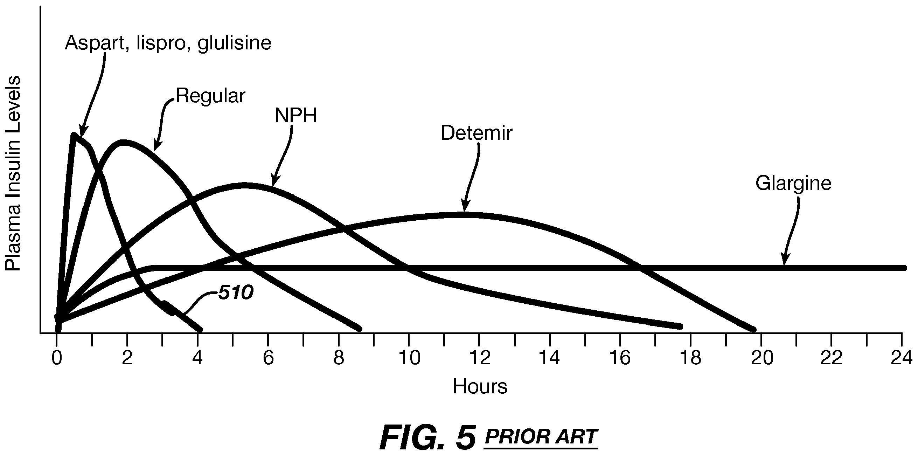

The effect of insulin action is subject to a time delay that depends on many factors, but the strongest dependency is on the type of insulin used. FIG. 5 shows time-action profiles of several types of insulin. Curves such as this one are generated when evaluating newly-diagnosed diabetic patients under a glucose clamp study, in order to determine their Insulin Sensitivity Factor (ISF).

## BRIEF DESCRIPTION OF THE INVENTION

Conventional insulin pumps use rapid acting insulins, such as NOVOLOG (aspart) and HUMALOG (lispro), regular insulin, or some mixture of them. Existing Model Predictive Control (MPC) models typically use differential or difference equations to represent a dynamic model, but they pick a standard insulin-action curve and keep it constant in their calculations.

The performance of MPC can be improved by adding a dynamic model of insulin time-action that adapts itself to the particular metabolism of each diabetic patient.

According to various aspects, there is provided a simple dynamic model for insulin time-action profiles. Various aspects are suitable for implementation in an embedded system such as an insulin pump, or a handheld device such as a pump remote control. Various aspects can be used as an off-line modeling tool running on a smartphone to assist patients in their treatment, or can be used as part of a closed-loop control algorithm for an artificial pancreas.

## BRIEF DESCRIPTION OF THE DRAWINGS

The accompanying drawings, which are incorporated herein and constitute part of this specification, illustrate various aspects, and, together with the general description given above and the detailed description given below, serve to explain features of various aspects (wherein like numerals represent like elements). The drawings are not necessarily to scale.

Figure 1 illustrates the system in which a controller for the pump or glucose monitor(s) is separate from both the infusion pump and the glucose monitor(s) and in which a network can be coupled to the controller to provide near real-time monitoring.

Figure 2 illustrates an exemplary embodiment of the diabetic management system in schematic form.

FIG. 3 is a high-level diagram showing the components of a data-processing system;

FIGS. 4 and 5 show examples of conventional insulin time-action curves;

FIG. 6 shows an exemplary modeled time-action curve according to various aspects;

FIG. 7 shows a parameterized Bayesian network useful for producing an adaptive model of insulin time-action profiles; and

FIG. 8 is a flowchart illustrating an exemplary method for controlling an insulin pump.

## DETAILED DESCRIPTION

The following detailed description should be read with reference to the drawings, in which like elements in different drawings are identically numbered. The drawings, which are not necessarily to scale, depict selected embodiments and are not intended to limit the scope of the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of aspects herein in a human patient are preferred. Furthermore, the term "user" includes not only the patient using a drug infusion device but also the caretakers (e.g., parent or guardian, nursing staff or home care employee). Users of a pump can adjust its settings for the benefit of a subject or patient. The term "drug" may include hormone, biologically active materials, pharmaceuticals or other chemicals that cause a biological response (e.g., glycemic response) in the body of a user or patient.

For controlling blood glucose, it is useful for users to measure their blood sugar, know the effect of insulin on blood sugar ("insulin sensitivity factor" or "ISF"), know how many grams of carbohydrates they are eating, know how much insulin is required for a given amount of carbohydrate ("insulin-to-carbohydrate ratio"), and know the effects of exercise and other activities on their blood sugar. Moreover, glucose measurements in the body show significant variability due to frequent changes in the glucose level and variability in the measurement instruments.

The curves shown in FIG. 5 can be fit with a variety of mathematical functions. In various aspects, a function is selected that can fit a range of curves for different insulin types by adjusting function parameters rather than the function itself. This permits more compact function implementations, which is particularly useful for embedded systems such as insulin pumps. In an aspect, a Beta distribution is used. In various aspects, a function that is not a differential equation is used in order to simplify its implementation on an embedded system, such as an insulin pump.

In various aspects, insulin time-action profiles (e.g., of regular or rapid-acting insulins) are approximated by the following lognormal function:

$$GIR(t) = at^{-1}e^{-b[\log(t)-c]^2} \qquad (A)$$

Where **GIR** stands for glucose infusion rate, **t** is time and **a, b, c** are parameters. This function features an inflection point before the peak, as do various measured insulin curves. The notation $\log(t)$ refers to the natural (base-$e$) logarithm, as do all logarithms described in this section. The function $e^x$ is also denoted $\exp(x)$.

In this model, the lognormal function parameters are related to physiological variables as described following.

Parameter **a** affects the amplitude of the peak directly, and it is a function **f** of the last dose of insulin, carbohydrate content of a meal, the patient's nominal Insulin Sensitivity Factor (*Isens*), and even perhaps physical activity and heart rate too:

$$a = f(LastDose, Carb, Isens, HeartRate, Activity)$$

Parameter **b** has some influence in the peak and the time to peak, and can be a function **g** of the carbohydrate **C** and fat **F** content of meals:

$$b = g(Carb, Fat)$$

Parameter c affects the morphology of the curve and it correlates to the characteristic time-action curves of specific insulins. Therefore, it is a constant that depends solely on the type of insulin used. In the case of HUMALOG, c=4.5.

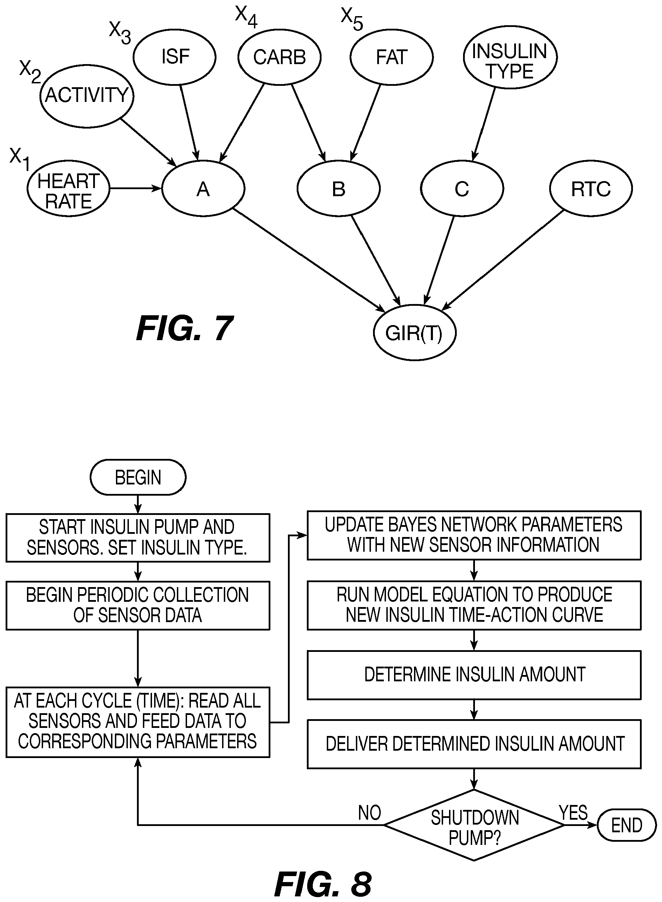

Various physiological variables can be measured; examples are described below with reference to FIG. 7. In some aspects, glucose level is not one of the physiological variables measured to determine *GIR(t)*.

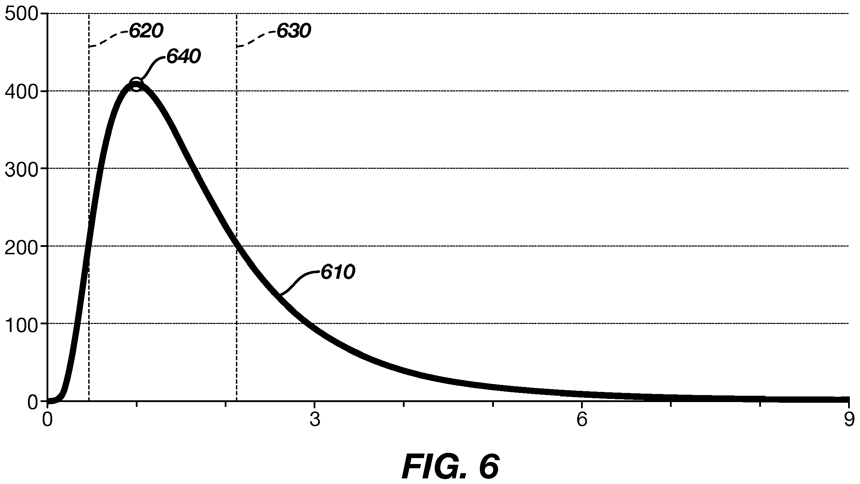

FIG. 6 shows as an example of a modeled time-action profile generated with this model that is similar to the time-action behavior of HUMALOG insulin published by its manufacturer. The model uses eq. (A), above, with the following parameter values: $a = 30000$ ; $b = 1.2$ ; $c = 4.5$. Curve 610 is the time-action curve. HUMALOG is a lispro insulin product; curve 610 is similar in shape to lispro curve 510 (FIG. 5).

This model has some additional useful characteristics. For example, the maximum glucose concentration that the insulin will cover at its peak is given by:

$$GIR_{max} = a \cdot exp\left(\frac{1}{4b} - c\right)$$

The time delay for the peak, after infusion, is determined by:

$$t_{max} = exp\left(c - \frac{1}{2b}\right)$$

The total glucose that the insulin infused at $t = 0$ will cover is found by integrating Eq. 1, also known as the area under the curve (AUC):

$$AUC_{0-\infty} = a \int_0^\infty t^{-1} e^{-b[\log(t)-c]^2} dt$$

$$AUC_{0-\infty} = a \sqrt{\frac{\pi}{b}}$$

Another property of curve 610 is:

$$t_{early50\%} = exp\left(c - \frac{1}{2b} - \sqrt{\frac{\log(2)}{b}}\right)$$

which is the time at which $GIR(t)$ has reached half of its maximum value 640 while increasing. This is shown on the figure by marker 620. Similarly,

$$t_{late50\%} = exp\left(c - \frac{1}{2b} + \sqrt{\frac{\log(2)}{b}}\right)$$

is the time at which $GIR(t)$ has reached half of its maximum value 640 while decreasing. This is shown on the figure by marker 630. As mentioned above, all logarithms in the preceding equations are natural logarithms.

Having physiological and meal information embedded directly into the insulin action model permits producing a personalized model that is updated, e.g., continuously, as the system gathers information about its environment. This represents a major advantage over existing solutions, as it continually refines itself, adapting over time to each individual patient.

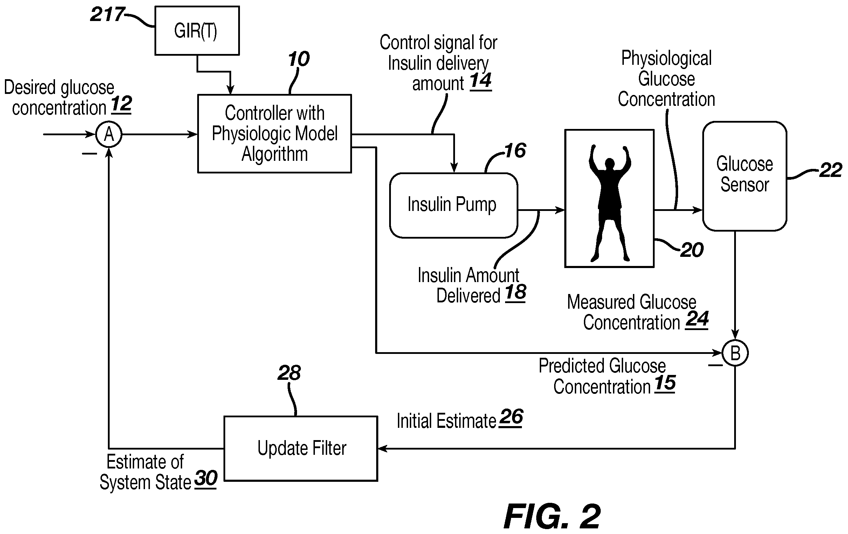

FIG. 7 shows a parameterized Bayesian network with real-time data feeding into the model parameters (a, b, c) to generate the adaptive model of insulin time-action profiles. The Real Time Clock (RTC) provides a time base for the model. This Bayesian network can be used to update model parameters according to various aspects. These parameters can then be used in an insulin control loop such as that discussed herein with reference to FIG. 2.

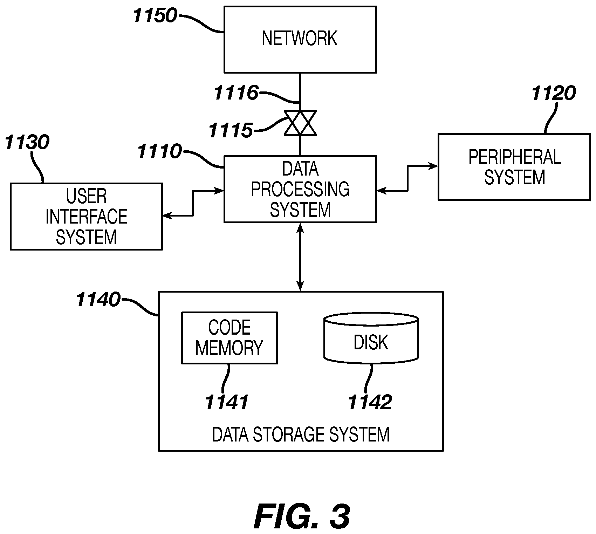

A Bayesian network is a directed acyclic graph with nodes representing random variables with values described by a probability distribution, which accounts for the inherent uncertainty of the environment. Physiological variables $X_1$, $X_2$, $X_3$, $X_4$, $X_5$ can be measured or provided directly by the subject or another person, e.g., by entering information into an interface for a portable insulin pump. $X_1$ is heart rate, $X_2$ is the amount of physical activity the subject is performing, $X_3$ is the subject's insulin sensitivity factor, and $X_4$ and $X_5$ are the carbohydrate and fat content, respectively, of a meal eaten by the subject. "INSULIN TYPE" is the type of insulin being delivered to the subject, used for determining parameter "C," and can be provided by, e.g., a menu selection on the interface (user interface system 1130, FIG. 3). Some of the sensed variables can be continuous with a Gaussian distribution over a specified domain, e.g., the patient's heart rate ($X_1$), insulin sensitivity factor ISF ($X_3$), and meal contents (Carb $X_4$, Fat $X_5$). Some can be discrete multivalued variables, including the patient activity indicator, e.g., Activity $X_2$ = None, Light, Moderate, or Vigorous; or Insulin type = Regular, HUMALOG, or NOVOLOG. Insulin Type could also have other values for various insulin mixtures.

Various aspects use machine-learning techniques to determine parameter values of the glucose infusion rate (GIR) model function. For example, Bayesian inference can be used for performing machine learning. In general, a Bayesian inference system attempts to infer a function that maps a set of input values (the input vector) to a set of output values (the output vector). In the Bayesian approach, one does not select a single "best" set of weights and biases for the neural network. Instead, one integrates the predictions from all possible weight and bias vectors over a posterior weight distribution that combines information from the data with a prior bias toward more plausible weight vectors. Thus, instead of outputting a specific single output result for a given input, a Bayesian network outputs a probability distribution of the result. To generate a specific result, one may integrate over the probability distribution and thus select the mean as a specific result. In various aspects, Parameters $X_1$, $X_2$, $X_3$, $X_4$, $X_5$, determine the posterior probability distribution of their corresponding variables. These parameters are continuously refined by updating them at each cycle as part of a Bayesian learning process. The Normal-Wishart distribution determines the conjugate prior for the parameters of a posterior Gaussian distribution (described in Bernardo and Smith. Bayesian Theory. Chichester: John Wiley & Sons Ltd., 2000. ISBN 0-471-49464-X), and the Dirichlet distribution determines the conjugate prior for the parameters of a posterior discrete multivalued distribution.

FIG. 8 is a flowchart illustrating an exemplary method for controlling an insulin pump. At the beginning of the process, the insulin pump and sensors are started and the insulin type is set. The insulin type can be, e.g., received from user interface system 1130 (FIG. 3), as described above with reference to FIG. 8. Once the pump and sensors are running, sensor data begins to be collected periodically, e.g., every five minutes. Sensor data can include blood glucose level or the level of another physiological fluid of a subject. Sensor data can also include data for heart rate and other variables discussed below.

Each time interval, e.g., every five minutes, readings from the sensors are taken and provided to a controller, e.g., data processing system 1110 (FIG. 11). The sensor readings can be staggered throughout the time interval or can be taken simultaneously or near-simultaneously. The controller then updates parameters of the Bayesian network (e.g., FIG. 7) based on the new sensor information. Time intervals can be evenly spaced or not; intervals can be skipped; the series of time intervals can be discontinuous.

The Bayesian network outputs new parameters (e.g., a, b, and c, FIG. 7) that feed a *GIR*(*t*) model such as that described herein. The model equation is run to produce a new insulin time-action curve. The time-action curve is used to determine an amount of insulin to be delivered to the subject. This is done by adjusting glucose-insulin model parameters based on the new insulin time-action curve, and then using the adjusted glucose-insulin model in the cost computation for zone MPC, as described below.

Bayesian inference and Bayesian learning, or other machine-learning techniques, can be used to determine parameters a, b, and c of the GIR model based on measurements or externally-provided data. Further details of Bayesian inference are given in U.S. Patent No. 6,883,148 to Teig et al.

For example, a Bayesian network may be trained on a known set of training data D where D={($X_i$, $Y_i$): i=1 to n) to build a Bayesian network model B(X). The Bayesian network B(X) outputs a probabilistic distribution p(Y|X)=B(X) for a given value novel input vector X. Specifically, the Bayesian network outputs an approximation of a probabilistic distribution p(Y|X) based on the training data D. There are both statistical and computational reasons as to why it is an approximation. The approximation may be referred to as p(Y|X, D) to indicate that it is dependent on the specific training data D.

A particular Bayesian model will be referred to as model H. Each Bayesian model H may be further defined by an m dimensional model parameter vector W that specific parameters of that particular model H. For example, in various neural network models, the parameter vector W defines the weights ($u_{ij}$ and $v_{jk}$) and biases ($a_j$ and $b_k$) for the neural network.

The Bayesian model H specifies a probability distribution function f(X, W) in terms of the input vector X and the model parameter vector W. Bayesian inference starts from "prior knowledge" (referred to simply as a "prior"), which is then updated in the light of the training data, giving rise to the posterior distribution p(Y|X, W). The prior is intended to capture our expectations about the model parameters, before we have seen any training data.

The prior knowledge may be formulated as a probability distribution over the quantities with which the Bayesian inference is concerned. The prior probability distribution for the m dimensional parameter vector W is p(W|H). Priors are often specified as functions of variables, $\alpha$, called "hyperparameters". Thus, a prior probability distribution dependent on the hyperparameters $\alpha$ can be specified as p(W|$\alpha$, H). (The hyperparameters $\alpha$ may be a single value or a vector.) Prior information about the value of a hyperparameter can also be expressed as a "hyperprior" which states the expectations about the value of the hyperparameter $\alpha$.

The data dependent term is given in a probabilistic term known as likelihood. Specifically, the likelihood defines the probability of a particular output value given the input data X, the model parameters W, and the model H. The likelihood of a particular output value Y can be expressed as p(Y|X,W,H).

Using Bayes' Rule, the posterior probability density of model parameters W conditioned on the hyperparameters $\alpha$ can be defined as:

$$p(W \mid \alpha, D, H) = \frac{p(Y \mid X, W, H) p(W \mid \alpha, H)}{p(Y \mid X, \alpha, H)}$$

To get rid of the hyperparameters $\alpha$, the posterior probability density of model parameters W may be integrated with respect to the posterior distribution of the hyperparameters

$$p(W|D,H) = \int p(W|\alpha, D, H) p(\alpha|D, H) d\alpha \qquad (B4)$$

The posterior distribution of the hyperparameters $\alpha$ can be obtained using Bayes' rule:

$$p(\alpha \mid D, H) = \frac{p(Y \mid X, \alpha, H) p(\alpha \mid H)}{p(Y \mid X, H)}$$

where the likelihood for the hyperparameters $\alpha$ is given by

$$p(Y|X,\alpha,H) = \int p(Y|X,W,H) p(W|\alpha, H) d^m W$$

As previously set forth, the Bayesian inference system does not give a particular output for a given model with defined model parameters. Instead, it outputs a probability distribution over the parameter space. To use a Bayesian model to make predictions, one must integrate over the posterior distribution of the model parameters given in equation (B4). Thus, the predictive probability distribution for

$$p(Y|X,D,H) = \int p(Y|X,W,D,H) p(W|D,H) d^m W \qquad (B7)$$

To make a specific prediction, one may integrate over the probability distribution to obtain a mean value of the predictive probability distribution. Thus, the predicted mean would be

$$\hat{y} = \int y p(y|X,D,H)$$

Detailed information on using Bayesian inference to train neural networks can be found in the paper "Bayesian Learning in Feed Forward Neural Networks" by Carl Edward Rasmussen of the Department of Computer Science at the University of Toronto.

## Monte Carlo Method Using Metropolis

Symbolic evaluation of the inferences made in the previous section on Bayesian learning is generally not possible. Thus, numerical computation is needed to evaluate the various complex integrals used in Bayesian learning.
Even using numerical computation, it is difficult to perform Bayesian learning for a neural network. Specifically, the very high dimensions of the integral in equation (B7) for complex models becomes quiet unwieldy. To simplify the computation, one may use random sampling techniques. Such techniques are often referred to as the "Monte Carlo" method in reference to the famous casino resort. It is difficult to sample the large solution space such that the sampling technique must actively search for regions with high probability.
The basic sampling technique is to approximate an integral over a function multiplied by a probability function by determining the mean of the function when sampled from the probability distribution. Thus, for n sufficiently large:

$$\int f(x)p(x)dx \cong \frac{1}{n}\sum_{i=1}^{n} f(x_i)$$

$$(B9)$$

where the vectors $x_i$ are randomly drawn from the probability distribution p(x).

There are a number of different methods of implementing Monte Carlo techniques for integration purposes. One well-known method is the Metropolis algorithm that employs Markov Chains in the Monte Carlo method. Fundamentals of the Metropolis algorithm can be found in the paper "Equation of state calculations by fast computing machines" by N. Metropolis, A. W. Rosenbluth, M. N. Rosenbluth, A. H. Teller, and E. Teller, in the "Journal of Chemical Physics", volume 21, pages 1087 to 1092.

The Metropolis algorithm was originally proposed as a method to simulate a system in a heat bath progressing toward thermal equilibrium. In the Metropolis algorithm, the system generates a new proposed state j of potential energy $E_j$ from a given current state i of potential energy $E_i$, by a small change in the system. If the new proposed state j has a smaller potential energy than the initial state I, then make state j the new current state, otherwise the system accepts state j with a probability of:

$$A_{ij}(T)dx = e^{-\frac{(\varepsilon_j - \varepsilon_i)}{kT}}$$

where k is a constant and T is the temperature of the heat bath. After a large number of iterations, the states visited by the algorithm forms an ergodic Markov Chain with a canonical distribution as the stationary distribution for the chain.

In one embodiment where $X_t$ defines the state of the system at time t, the one step transition probabilities for the Metropolis algorithm are:

$$p_{ij}(T) = P[X_{t+1} = j \mid X_t = i] = \begin{cases} G_{ij}(T)A_{ij}(T) & \text{if } i \neq j \\ 1 - \sum_{k \neq j} p_{ij}(T) & \text{if } i = j \end{cases}$$

where

- $G_{ij}(T)$=probability of generating j from i
- $A_{ij}(T)$=probability of accepting j from i

Referring back to equation (B9), the Metropolis algorithm can be used to generate a Markov chain of vectors $x_i$ in order to use equation (B9) to evaluate a difficult integral. Specifically, a candidate vector {tilde over (x)}$_{t+1}$ is generated for each iteration t by picking the vector according to some distribution p({tilde over (x)}$_{t+1}$|x$_t$). The candidate vector {tilde over (x)}$_{t+1}$ is accepted if it has lower energy than the previous state; if it has higher energy, it is accepted with a probability of $e^{-(E_{t+1}-E_d)}$. Stated formally:

$$x_{t+1} = \begin{cases} \tilde{x}_{t+1} & \text{if random } [0,1) < e^{-(E_{t+1}-E_{st})} \\ x_t & \text{otherwise} \end{cases}$$

$$(B12)$$

Thus, using the vector selection of equation (B12), a Markov chain of vectors may be generated to numerically solve integrals using the Monte Carlo method.

Although the Monte Carlo method implemented with the Metropolis algorithm works reasonably well, it make take a very large number of iterations to accurately numerically solve integrals using the Metropolis algorithm. This is because the Metropolis algorithm essentially performs a "random walk" through the solution space with very small steps. It would be desirable to have a system that is faster at finding a good solution.

**Hybrid Monte Carlo Method**

To find a good solution more rapidly, a Hybrid Monte Carlo technique may be used. A Hybrid Monte Carlo technique takes advantage of gradient information provided by backpropagation networks in order to guide the search toward solutions that have a high probability of being accepted.

The Hybrid Monte Carlo technique considers both "kinetic" energy and potential energy instead of just the potential energy considered by the Metropolis algorithm. Thus, Hamiltonian mechanics are used. To represent the overall state of a system, the Hybrid Monte Carlo technique uses two vectors: a position state vector Q (used to determine the potential energy in the system) and a momentum state vector K (used to determine the kinetic energy of the system). The overall energy of the system is defined by adding both the potential energy and the kinetic energy. Specifically, the overall energy of the system [H(Q, K)] is defined by

$$H(Q, K) = E(Q) + \frac{1}{2}|K|^2$$

To iterate the using the Hybrid Monte Carlo technique, one must generate a Markov chain of vectors $(Q_0, K_0)$, $(Q_1, K_1)$, $(Q_2, K_2)$, etc. The Markov chain is generated using two types of transitions: "dynamic" moves that explore the surfaces over which H is constant and "stochastic" moves that explore states with different values of H with probabilities proportional to $e^{-H}$ One method of changing H is to replace the momentum vector K with one drawn from the stationary momentum distribution:

$$p(K) = (2\pi)^{-\frac{N}{2}} e^{-\frac{1}{2}|K|^2}$$

The dynamic moves follow Hamilton's equations. Specifically, Hamilton's equations define derivatives of Q and K with respect to a time variable $\tau$ as:

$$\frac{dQ}{d\tau} = +\frac{\partial H}{\partial K} = K$$

$$\frac{dK}{d\tau} = -\frac{\partial H}{\partial Q} = -\nabla E(Q)$$

In one embodiment, the system generates a proposed state ({tilde over (Q)}$_{t+1}$, {tilde over (K)}$_{t+1}$) by negating the momentum vector K with a probability of 0.5, then following the above Hamilton dynamics for a time period, and again negating the momentum vector K with a probability of 0.5. Other embodiments may generate proposed vectors in other means.

The generated proposed state ({tilde over (Q)}$_{t+1}$, {tilde over (K)}$_{t+1}$) is then accepted in a manner similar to the Metropolis algorithm. Specifically, the generated proposed state ({tilde over (Q)}$_{t+1}$, {tilde over (K)}$_{t+1}$) is accepted as follows:

$$(Q_{t+1}, K_{t+1}) = \begin{cases} (\tilde{Q}_{t+1}, \tilde{K}_{t+1}) & \text{if random } [0, 1) < e^{-\Delta H} \\ (Q_t, K_t) & \text{otherwise} \end{cases}$$

Details on the Hybrid Monte Carlo technique can be found in the paper titled "Hybrid Monte Carlo", by S. Duane, A. D. Kennedy, B. J. Pendleton, and D. Roweth, in Physics Letters B, volume 195, pages 216 to 222.

To implement the Hybrid Monte Carlo technique in a discrete digital environment, one may use the "leapfrog" method. The leapfrog method discretizes Hamilton's equations using a non-zero step size $\varepsilon$ as follows:

$$K\left(\tau + \frac{\varepsilon}{2}\right) = K(\tau) - \frac{\varepsilon}{2} \nabla E(Q(\tau))$$

$$Q(\tau + \varepsilon) = Q(\tau) + \varepsilon K\left(\tau + \frac{\varepsilon}{2}\right)$$

$$K(\tau + \varepsilon) = K\left(\tau + \frac{\varepsilon}{2}\right) - \frac{\varepsilon}{2} \nabla E(Q(\tau + \varepsilon))$$

These equations may be iterated a number of times to generate a proposed state $(\tilde{Q}_{t+1}, \tilde{K}_{t+1})$. The greater the number of iterations that are performed, the faster the space will be explored. However, if too many iterations are performed, the rejection rate may become too high.

The following pseudocode illustrates a sample implementation of the Hybrid Monte Carlo method using the leapfrog method:

```
  # x is the current position vector
g = gradE (x); # set gradient using initial x
E = findE (x); # set objective energy function
for 1 = 1:L # loop L times
  p=randn (size (x)); # initial momentum = Normal(O,1)
  H = E + (|p|**2)/; # evaluate current energy H(x,p)
  xnew = x; # Start from current position
  gnew = g; # and current gradient
  # Perform leapfrog steps using equations (18), (19) and (20)
  for t = 1:Tau # make Tau leapfrog steps
     p = p - epsilon*gnew/2; # make ½ step in p eq(18)
     xnew = xnew + epsilon*p; # make step in x eq(19)
     gnew = gradE(xnew); # find new gradient for eq(20)
     p = p - epsilon*gnew/2; # make ½ step in p eq(20)
  endfor
  Enew = findE(xnew); # find new potential energy
  Hnew = (|p|**2)/2 + Enew; # find new value of H
  DeltaH = Hnew - H; # Determine energy difference
  # Decide whether to accept using equation (17)
  if (rand( ) < exp(-DeltaH))
          # Proposed state accepted, thus ...
     x = xnew; # Set current position x to xnew
     g = gnew; # Set current gradient g to gnew
     E = Enew; # Set current energy E to Enew
  endif
endfor
```

Details on implementing the Hybrid Monte Carlo technique in a neural network can be found in the paper "Bayesian Training of Backpropagation Networks by the Hybrid Monte Carlo Method" by Radford M. Neal of the Department of Computer Science at the University of Toronto in Technical Report CRG-TR-91-1 of the Connectionist Research Group (1992) and in Radford M. Neal's 1995 PhD thesis "Bayesian Learning for Neural Networks" for Department of Computer Science at the University of Toronto. Additional useful information may be found in the paper entitled "A Practical Monte Carlo Implementation of Bayesian Learning" by Carl Edward Rasmussen of the Department of Computer Science at the University of Toronto.

Additional information about machine learning is given in U.S. Patent No. 6,857,112 to Teig. Additional information about machine learning is given in U.S. Publication Nos. US20030088320A1, US20030232314A1, US20040131107A1, US20040250166A1, US20050105682A1, US20050123893A1, US20070091085A1, US20080052312A1, US20080065471A1, US20080208581A1, US20090004638A1, US20090144101A1, US20090144123A1, US20090161741A1, US20090279737A1, US20100076913A1, US20100135584A1, US20100161611A1, US20100262568A1, US20100280804A1, US20110087673A1, US20110153419A1, US20110206246A1, US20110231704A1, US20110256607A1, US20110302031A1, US20110314039A1, US20120059253A1, US20120143789A1, US20120155717A1, US20120202212A1, US20120271556A1, and in U.S. Patent Nos. US6687887B1, US6735748B1, US6832069B2, US6857112B1, US6883148B1, US6892366B1, US6907591B1, US7051293B1, US7085690B2, US7099435B2, US7103524B1, US7213174B2, US7457581B2, US7684651B2, US7755619B2, US7756682B1, US7860347B2, US7865336B1, US7877234B1, US7974570B2, US8090665B2, US8170905B2, US8204838B2, US8229165B2, US8234274B2, US8280705B2, US8301482B2.

In view of this, various aspects dynamically model insulin time-action profiles. A technical effect is to provide improved glucose-infusion-rate model parameters using Bayesian-network machine-learning techniques. These improved model parameters can be used to predict the blood glucose level of a subject, as described more fully below. This, in turn, permits more accurately controlling the blood glucose level of a diabetic patient using an insulin pump.

In various aspects, features described herein may also be utilized in combination. For example, the at least one glucose sensor may include both a continuous glucose sensor and an episodic glucose meter

In various aspects, a system for management of diabetes is provided that includes a continuous glucose meter and an infusion pump coupled to a controller. The continuous glucose monitor continuously measures glucose level of the subject at discrete generally uniform time intervals and provides the glucose level at each interval in the form of glucose measurement data. The insulin infusion pump is controlled by the controller to deliver insulin to the subject. The controller is in communication with the pump, glucose meter and the glucose monitor.

In each of the above aspects, the following features may also be utilized in combination with each of the aspects. For example, the at least one glucose sensor may include both a continuous glucose sensor and an episodic glucose meter.

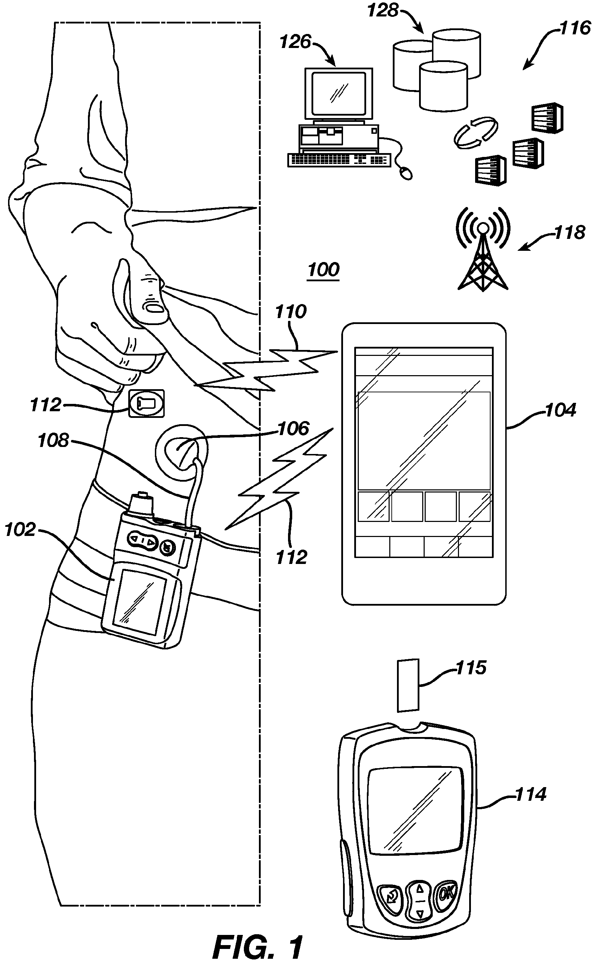

FIG. 1 illustrates a drug delivery system 100 according to an exemplary embodiment. Drug delivery system 100 includes a drug delivery device 102 and a remote controller 104. Drug delivery device 102 is connected to an infusion set 106 via flexible tubing 108.

Drug delivery device 102 is configured to transmit and receive data to and from remote controller 104 by, for example, radio frequency communication 112. Drug delivery device 102 may also function as a stand-alone device with its own built in controller. In one embodiment, drug delivery device 102 is an insulin infusion device and remote controller 104 is a hand-held portable controller. In such an embodiment, data transmitted from drug delivery device 102 to remote controller 104 may include information such as, for example, insulin delivery data, blood glucose information, basal, bolus, insulin to carbohydrates ratio or insulin sensitivity factor, to name a few. The controller 104 is configured to include an MPC controller 10 that has been programmed to receive continuous glucose readings from a CGM sensor 112. Data transmitted from remote controller 104 to insulin delivery device 102 may include glucose test results and a food database to allow the drug delivery device 102 to calculate the amount of insulin to be delivered by drug delivery device 102. Alternatively, the remote controller 104 may perform basal dosing or bolus calculation and send the results of such calculations to the drug delivery device. In an alternative embodiment, an episodic blood glucose meter 114 may be used alone or in conjunction with the CGM sensor 112 to provide data to either or both of the controller 104 and drug delivery device 102. Alternatively, the remote controller 104 may be combined with the meter 114 into either (a) an integrated monolithic device; or (b) two separable devices that are dockable with each other to form an integrated device. Each of the devices 102, 104, and 114 has a suitable micro-controller (not shown for brevity) programmed to carry out various functionalities.

Drug delivery device 102 may also be configured for bi-directional wireless communication with a remote health monitoring station 116 through, for example, a wireless communication network 118. Remote controller 104 and remote monitoring station 116 may be configured for bi-directional wired communication through, for example, a telephone land based communication network. Remote monitoring station 116 may be used, for example, to download upgraded software to drug delivery device 102 and to process information from drug delivery device 102. Examples of remote monitoring station 116 may include, but are not limited to, a personal or networked computer 126, server 128 to a memory storage, a personal digital assistant, other mobile telephone, a hospital base monitoring station or a dedicated remote clinical monitoring station.

Drug delivery device 102 includes electronic signal processing components including a central processing unit and memory elements for storing control programs and operation data, a radio frequency module 116 for sending and receiving communication signals (i.e., messages) to/from remote controller 104, a display for providing operational information to the user, a plurality of navigational buttons for the user to input information, a battery for providing power to the system, an alarm (e.g., visual, auditory or tactile) for providing feedback to the user, a vibrator for providing feedback to the user, a drug delivery mechanism (e.g. a drug pump and drive mechanism) for forcing a insulin from a insulin reservoir (e.g., a insulin cartridge) through a side port connected to an infusion set 108/106 and into the body of the user.

Glucose levels or concentrations can be determined by the use of the CGM sensor 112. The CGM sensor 112 utilizes amperometric electrochemical sensor technology to measure glucose with three electrodes operably connected to the sensor electronics and are covered by a sensing membrane and a biointerface membrane, which are attached by a clip.

The top ends of the electrodes are in contact with an electrolyte phase (not shown), which is a free-flowing fluid phase disposed between the sensing membrane and the electrodes. The sensing membrane may include an enzyme, e.g., glucose oxidase, which covers the electrolyte phase. In this exemplary sensor, the counter electrode is provided to balance the current generated by the species being measured at the working electrode. In the case of a glucose oxidase based glucose sensor, the species being measured at the working electrode is $H_2O_2$. The current that is produced at the working electrode (and flows through the circuitry to the counter electrode) is proportional to the diffusional flux of $H_2O_2$. Accordingly, a raw signal may be produced that is representative of the concentration of glucose in the user's body, and therefore may be utilized to estimate a meaningful glucose value. Details of the sensor and associated components are shown and described in US Patent No. 7276029. In one embodiment, a continuous glucose sensor from the DEXCOM Seven System® (manufactured by DEXCOM Inc.) can also be utilized with the exemplary embodiments described herein.

In one embodiment, the following components can be utilized as a system for management of diabetes that is akin to an artificial pancreas: OneTouch Ping® Glucose Management System by Animas Corporation that includes at least an infusion pump and an episodic glucose sensor; and DexCom® SEVEN PLUS® CGM by DEXCOM Corporation with interface to connect these components and programmed in MATLAB® language and accessory hardware to connect the components together; and control algorithms in the form of an MPC that automatically regulates the rate of insulin delivery based on the glucose level of the patient, historical glucose measurement and anticipated future glucose trends, and patient specific information.

FIG. 2 illustrates a schematic diagram 200 of the system 100 in FIG. 1 programmed with the solution devised by applicants to counteract a less than desirable effect of a closed-loop control system. In particular, FIG. 2 provides for an MPC programmed into a control logic module 10 that is utilized in controller 104. MPC logic module 10 receives a desired glucose concentration or range of glucose concentration 12 (along with any modification from an update filter 28 so that it is able to maintain the output (i.e., glucose level) of the subject within the desired range of glucose levels. MPC logic module 10 also receives parameters derived from function 217, *GIR(t),* as described herein.

Referring to FIG. 2, the first output 14 of the MPC-enabled control logic 10 can be a control signal to an insulin pump 16 to deliver a desired quantity of insulin 18 into a subject 20 at predetermined time intervals, which can be indexed every 5 minutes using time interval index k. A second output in the form of a predicted glucose value 15 can be utilized in control junction B. A glucose sensor 22 (or 112 in Fig. 1) measures the glucose levels in the subject 20 in order to provide signals 24 representative of the actual or measured glucose levels to control junction B, which takes the difference between measured glucose concentration 24 and the MPC predictions of that measured glucose concentration. This difference provides input for the update filter 26 of state variables of the model. The difference 26 is provided to an estimator (also known as an update filter 28) that provides for estimate of state variables of the model that cannot be measured directly. The update filter 28 is preferably a recursive filter in the form of a Kalman filter with tuning parameters for the model. The output of the update or recursive filter 28 is provided to control junction A whose output is utilized by the MPC in the control logic 10 to further refine the control signal 14 to the pump 16 (or 102 in Fig. 1).

A brief overview of the MPC noted above that is used in control logic 10 is warranted here. The MPC logic is formulated to control a subject glucose level to a safe glucose zone, with the lower blood glucose limit of the zone varying between 80-100 mg/dL and the upper blood glucose limit varying between about 140-180 mg/dL; the algorithm will henceforth be referred to as the "zone MPC". Controlling to a target zone is, in general, applied to controlled systems that lack a specific set point with the controller's goal being to keep the controlled variable, (CV), for example the glucose values, in a predefined zone. Control to zone (i.e., a euglycemic zone) is highly suitable for the artificial pancreas because of the absence of a natural glycemic set point. Moreover, an inherent benefit of control to zone is the ability to limit pump actuation/activity in a way that if glucose levels are within the zone then no extra correction shall be suggested.

In real-time, the insulin delivery rate $I_D$ from the zone MPC law is calculated by an on-line optimization, which evaluates at each sampling time the next insulin delivery rate. The optimization at each sampling time is based on the estimated metabolic state (plasma glucose, subcutaneous insulin) obtained from the dynamic model stored in module 10.

The MPC of control logic 10 incorporates an explicit model of human T1DM glucose-insulin dynamics. The model is used to predict future glucose values and to calculate future controller moves that will bring the glucose profile to the desired range. MPC in controllers can be formulated for both discrete- and continuous-time systems; the controller is set in discrete time, with the discrete time (stage) index $k$ referring to the epoch of the $k^{th}$ sample occurring at continuous time $t = k \cdot T_s$, where $T_s$ = 5 min is the sampling period. Software constraints ensure that insulin delivery rates are constrained between minimum (i.e., zero) and maximum values. The first insulin infusion (out of $N$ steps) is then implemented. At the next time step, $k+1$ based on the new measured glucose value and the last insulin rate, the process is repeated.

Specifically, we start with the original linear difference model used for zone MPC:

$$G'(k) = a_1 G'(k-1) + a_2 G'(k-2) + a_3 G'(k-3) + a_4 G'(k-4) + a_5 G'(k-5) + bI_M(k-4)$$

$$I_M(k) = c_1 I_M(k-1) + c_2 I_M(k-2) + d_1 I'_D(k-1) + d_2 I'_D(k-2)$$

$$\text{Eq. (1)}$$

where:

k    is the discrete time interval index having a series of indexing counters where k=1, 2, 3 ...

G'    is the measured glucose concentration

$I_M$    is the "mapped insulin" which is not a measured quantity

$I'_D$    is the delivered insulin or a manipulated variable

and coefficients $a_1 \sim 2.993$; $a_2 \sim (-3.775)$; $a_3 \sim 2.568$; $a_4 \sim (-0.886)$; $a_5 \sim 0.09776$; $b \sim (-1.5)$; $c_1 \sim 1.665$; $c_2 \sim (-0.693)$; $d_1 \sim 0.01476$; $d_2 \sim 0.01306$.

These coefficients represent a model of the pharmacokinetics and pharmacodynamics of insulin. Insulin level changes over time according to the $c_n$ and $d_n$ coefficients, and glucose level changes over time according to insulin level, the $a_n$ coefficient, and the $b$ coefficient.

Using the FDA accepted metabolic simulator known to those skilled in the art, Eq. (1) can be reduced to the following linear difference model in Equation (2):

$$(a) \quad G'(k) = \quad 2.993G'(k-1) - 3.775G'(k-2) + 2.568G'(k-3) - 0.886G'(k-4)$$
$$+ 0.09776G'(k-5)$$
$$- 1.5I_M(k-4)$$
$$+ 0.1401Meal_M(k-2) + 1.933Meal_M(k-3)$$

$$(b) \quad I_M(k) = \quad 1.665I_M(k-1) - 0.693I_M(k-2)$$
$$+ 0.01476I_D'(k-1) + 0.01306I_D'(k-2)$$

$$(2)$$

$$(c) \quad Meal_M(k) = \quad 1.501Meal_M(k-1) + 0.5427Meal_M(k-2)$$
$$+ 0.02279Meal(k-1) + 0.01859Meal(k-2)$$

where:

$G'$    is the glucose concentration output ($G$) deviation variable (mg/dL), i.e., $G' \equiv G$-110 mg/dL,

$I_D'$    is the insulin infusion rate input ($I_D$) deviation variable (U/h), i.e., $I_D' \equiv I_D$ - $basal$ U/h,

$Meal$    is the CHO ingestion input (gram- CHO),

$I_M$    is the mapped subcutaneous insulin infusion rates (U/h), and

$Meal_M$ is the mapped CHO ingestion input (gram- CHO).

The dynamic model in Eq. (2) relates the effects of insulin infusion rate ($I_D$), and CHO ingestion input (*Meal*) on plasma glucose. The model represents a single average model for the total population of subjects. In prior systems, the model and its parameters are fixed. According to various aspects described herein, the model or its parameters can change over time. The *GIR*(*t*) function can be decomposed into a form usable in a linear difference model, e.g., by taking *GIR*(*t*) - *GIR*(*t-1*) symbolically and performing algebraic manipulation to derive an iterative model of *GIR*. In general, body or other parameters are measured (CGM, heart rate, others), the model or its parameters are updated, model prediction and MPC calculation are performed, and insulin is delivered. These steps are then repeated for as long as desired. The MPC utilizes GIR(t) to determine the G' function, then evaluates G'(k) to obtain the predicted glucose response for time interval *k*. In various aspects, in order to determine a GIR(t) for use by the MPC, the properties of GIR developed above (e.g., $GIR_{max}$ and $t_{max}$) are used to guide the search for correct a, b, and c parameters.. Examples of determining glucose models from *GIR* and similar information are described in Kovatchev et al. "In Silico Preclinical Trials: A Proof of Concept in Closed-Loop Control of Type 1 Diabetes." J Diabetes Sci Technol. 2009 January; 3(1): 44-55.

The second-order input transfer functions described by parts (b) and (c) in Eq. (2) are used to generate an artificial input memory in the zone MPC schema to prevent insulin overdosing, and consequently prevent hypoglycemia. In order to avoid over-delivery of insulin, the evaluation of any sequential insulin delivery must take into consideration the past administered insulin against the length of the insulin action. However, a one-state linear difference model with a relatively low order uses the output (glycemia) as the main source of past administered input (insulin) "memory." In the face of the model mismatch, noise, or change in the subject's insulin sensitivity, this may result in under- or over-delivery of insulin. This is mitigated by adding two additional states ($I_M$ and $Meal_M$) for the mapped insulin and meal inputs that carry a longer insulin memory.

Zone MPC is applied when the specific set point value of a controlled variable (CV) is of low relevance compared to a zone that is defined by upper and lower boundaries. Moreover, in the presence of noise and model mismatch there is no practical value using a fixed set point. Zone MPC was developed through research by the University of California at Santa Barbara and the Sansum Diabetes Research Institute. Other details of the derivation for the Zone MPC technique are shown and described in Benyamin Grosman, Ph.D., Eyal Dassau, Ph.D., Howard C. Zisser, M.D., Lois Jovanovic, M.D., and Francis J. Doyle III, Ph.D. "Zone Model Predictive Control: A Strategy to Minimize Hyper and Hypoglycemic Events" Journal of Diabetes Science and Technology, Vol. 4, Issue 4, July 2010, and US Patent Application Publication No. 2011/0208156 to Doyle et al., entitled *"Systems, Devices, and Methods to Deliver Biological Factors or Drugs to a Subject*," with the publication date of August 25, 2011, all which are incorporated by reference. Additional details of the Zone MPC are shown and described in US Patent Application Publication No. 20110208156. A related derivation of zone MPC was presented in Maciejowski JM., "PREDICTIVE CONTROL, WITH CONSTRAINTS" Harlow, UK: Prentice-Hall, Pearson Education Limited, 2002. The zone MPC is implemented by defining fixed upper and lower bounds as soft constraints by letting the optimization weights switch between zero and some final values when the predicted CVs are in or out of the desired zone, respectively. The predicted residuals are generally defined as the difference between the CV that is out of the desired zone and the nearest bound. Zone MPC is typically divided into three different zones. The permitted range is the control target and it is defined by upper and lower bounds. The upper zone represents undesirable high predicted glycemic values. The lower zone represents undesirable low predicted glycemic values that represent hypoglycemic zone or a pre-hypoglycemic protective area that is a low alarm zone. The zone MPC optimizes the predicted glycemia by manipulating the near-future insulin control moves to stay in the permitted zone under specified constraints.

Model predictive control operates by mathematically minimizing a cost function. For example, future glucose levels are predicted from past glucose levels and insulin amounts and from the candidate insulin amounts to be delivered in the future, e.g., using linear difference models of insulin-glucose dynamics. A cost is assigned to these predicted glucose levels. For zone MPC, the cost function defines the zone of control by setting cost much lower (e.g., 0) within the zone than out of the zone. Therefore, the cost of future glucose levels causes the optimization to select future $I'_D$ values that will tend to keep the predicted outputs within the zone of control (e.g., a zone defined by upper and lower bounds), rather than future values that will move the predicted outputs towards a specific set point. Optimizing using such a cost function can reduce hypo- and hyper-glycemic excursions from the zone of control. The aggressiveness of the controller in reducing excursions is influenced by the cost function, e.g., weights contained therein.

The zone MPC cost function J used in various aspects is defined as follows:

$$J(I_D') = Q \cdot \sum_{j=1}^{P} \left\| G^{zone}(k+j) \right\| + R \cdot \sum_{j=0}^{M-1} \left\| I_D'(k+j) \right\|$$

$$s.t. \tag{3}$$

$$G(k+j) = f[G(k+j-1), I_D'(k+j-1)] \qquad \forall j = 1, P$$

$$-basal(k+j) \le I_D'(k+j) \le 72 \qquad \forall j = 0, M-1$$

or for various aspects described herein:

$$J(I_D') = \sum \left\| G^{zone}(k+j) \right\| + R \cdot \sum \left\| I_D(k+j) - basal(k+j) \right\| \tag{4}$$

where

Q is a weighting factor on the predicted glucose term;
R is a tuning factor on the future proposed inputs in the cost function;
f is the prediction function (in Eq. (2));
vector $I_D$ contains the set of proposed near-future insulin infusion amounts. It is the "manipulated variable" because it is manipulated in order to find the minimum in J.

$G^{zone}$ is a variable quantifying the deviation of future model-predicted CGM values G outside a specified glycemic zone, and is determined by making the following comparisons:

$$G^{zone} = \begin{cases} 0 & \text{if } G_{ZL} \le G \le G_{ZH} \\ G - G_{ZH} & \text{if } G > G_{ZH} \\ G_{ZL} - G & \text{if } G < G_{ZL} \end{cases} \tag{5}$$

where the glycemic zone is defined by the upper limit $G_{ZH}$ and the lower limit $G_{ZL}$.

Thus, if all the predicted glucose values are within the zone, then every element of $G^{zone}$ is equal to 0, and consequently J is minimized with $I_D = basal$ for that time of day, i.e., the algorithm "defaults" to the patient's current basal insulin infusion rate. On the other hand, if any of the predicted glucose values are outside the zone, then $G^{zone} > 0$ and thus "contributes" to the cost function. In this case, the near-future proposed insulin infusion amounts $I_D$ will deviate from the basal in order to prevent out-of-zone deviation in $G^{zone}$ from ever happening, which will also "contribute" to the cost function. Then, a quantitative balance is found in the optimization, based on the weighting factor R.

In order to solve optimization problem of Equations (2)-(5), a commercially available software routine (e.g., MATLAB's "fmincon.m" function) is utilized. For this function, the following parameters are used for each optimization:

∘ Initial guess for the insulin delivery rates, $I_D'(0)$, is the null vector $\vec{0} \in \mathbf{R}^M$, e.g., if M = 5 the initial guess for each optimization is $I_D' = [0\ 0\ 0\ 0\ 0]$. This implies that the initial guess is equivalent to the basal rate.
∘ Maximum number of function evaluations allowed is $Max\_f = 100*M$, where M is control horizon as described earlier.
∘ Maximum number of iterations is $Max\_i = 400$, which is fixed.
∘ Termination on the cost function values $Term\_cost = 1e-6$, which is fixed.
∘ Termination tolerance $Term\_tol$ on the manipulated variables $I_D'$ is 1e-6.

The following hard constraints are implemented on the manipulated variables ($I_D'$):

$$-basal \le I_D' \le 72 \text{ U/h} \tag{6}$$

where basal is the subject's basal rate as set by the subject or his/her physician, e.g., in the range 0.6 - 1.8 U/hr.
Although the values of control horizontal parameter M and prediction horizon parameter P have significant effects on the controller performance, and are normally used to tune an MPC based controller, they can be heuristically tuned based on knowledge of the system. Tuning rules are known to those skilled in the field. According to these rules *M* and *P* may vary between:

$$2 \leq M \leq 10$$
$$20 \leq P \leq 120 \tag{7}$$

In the preferred embodiments, we use the nominal values of *M* = 5 and *P* = 108.

The ratio of the output error weighting factor *Q* and the input change weighting matrix or tuning factor *R* may vary between:

$$10 \leq \frac{R}{Q} \leq 1000 \tag{8}$$

In the preferred embodiments, we use the nominal value of *R/Q* = 500.

Once the controller is initialized and switched on, real-time calculations take place every five minutes, corresponding to the sample time for the glucose sensor. The first element of $I_D$ is delivered as an insulin dose to the patient through the insulin pump, five minutes elapse, a new CGM reading becomes available, and the process repeats. It is noted that the future control moves are hard-constrained, set by the insulin pump's ability to deliver a maximum rate of insulin and the inability to deliver negative insulin values. Other details of related subject matter including state estimator, and other MPC are provided by Rachel Gillis et al., "Glucose Estimation and Prediction through Meal Responses Using Ambulatory Subject Data for Advisory Mode Model Predictive Control" Journal of Diabetes Science and Technology Vol. 1, Issue 6, Nov. 2007 and by Youqing Wang et al., "Closed-Loop Control of Artificial Pancreatic β-Cell in Type 1 Diabetes Mellitus Using Model Predictive Iterative Learning Control" IEEE Transactions on Biomedical Engineering, Vol. 57, No. 2, February 2010.

It is known that the tuning parameter (designated here as "R") can have a significant effect on the quality of the glucose control. The parameter - known as the aggressiveness factor, gain, and other names - determines the speed of response of the algorithm to changes in glucose concentration. A relatively conservative value of R results in a controller that is slow to adjust insulin infusion amounts (relative to basal) in response to changes in glucose; on the other hand, a relatively aggressive value of R results in a controller that is quick to respond to changes in glucose. In principle, an aggressive controller would result in the best glucose control if 1) the available glucose measurements are accurate, and moreover 2) the model predictions of future glucose trends are accurate. If these conditions are not true, then it may be safer to use a conservative controller.

To recap, the system of FIG. 2 is provided to manage diabetes of a subject. In this system, the following components are utilized: continuous glucose sensor 22, pump 16, and controller 10. The continuous glucose monitor continuously measure glucose level of the subject at discrete generally uniform time intervals (e.g., approximately every 30 seconds or every minute) and provide the glucose level at each interval in the form of glucose measurement data for each interval in a time interval index (k, where a time interval between k and k+1 is about 5 minutes). The insulin infusion pump is controlled by the controller 10 to deliver insulin to the subject 20. The controller 10 is programmed with the appropriate MPC program to control the pump and communicate with the glucose meter and the glucose monitor. In various aspects, the controller determines an insulin delivery rate for each time interval in the time interval index (k) from the model predictive control based on desired glucose concentration 12 and glucose concentration 24 measured by the monitor 22 at each interval of the interval index (k).

FIG. 3 is a high-level diagram showing the components of a data-processing system for analyzing data and performing other analyses described herein. The system includes a data processing system **1110,** a peripheral system **1120,** a user interface system **1130,** and a data storage system **1140.** The peripheral system **1120,** the user interface system **1130** and the data storage system **1140** are communicatively connected to the data processing system **1110.** Data processing system 1110 can be communicatively connected to network 1150, e.g., the Internet or an X.25 network, as discussed below. A controller for an insulin pump, e.g., controller 104 (FIG. 1) or a controller integrated with the pump, can include one or more of systems 1110, 1120, 1130, 1140, and can connect to one or more network(s) 1150. Data processing system 1110 in controller 104 can implement Bayesian learning, as described above, to determine GIR model parameters and use them in determining blood glucose and insulin infusion rate.

The data processing system **1110** includes one or more data processor(s) that implement processes of various aspects described herein. A "data processor" is a device for automatically operating on data and can include a central processing unit (CPU), a desktop computer, a laptop computer, a mainframe computer, a personal digital assistant, a digital camera, a cellular phone, a smartphone, or any other device for processing data, managing data, or handling data, whether implemented with electrical, magnetic, optical, biological components, or otherwise.

The phrase "communicatively connected" includes any type of connection, wired or wireless, between devices, data processors, or programs in which data can be communicated. Subsystems such as peripheral system **1120,** user interface system **1130,** and data storage system **1140** are shown separately from the data processing system **1110** but can be stored completely or partially within the data processing system **1110.**

The data storage system **1140** includes or is communicatively connected with one or more tangible non-transitory computer-readable storage medium(s) configured to store information, including the information needed to execute processes according to various aspects. A "tangible non-transitory computer-readable storage medium" as used herein refers to any non-transitory device or article of manufacture that participates in storing instructions which may be provided to data processing system 1110 for execution. Such a non-transitory medium can be non-volatile or volatile. Examples of non-volatile media include floppy disks, flexible disks, or other portable computer diskettes, hard disks, magnetic tape or other magnetic media, Compact Discs and compact-disc read-only memory (CD-ROM), DVDs, BLU-RAY disks, HD-DVD disks, other optical storage media, Flash memories, read-only memories (ROM), and erasable programmable read-only memories (EPROM or EEPROM). Examples of volatile media include dynamic memory, such as registers and random access memories (RAM). Storage media can store data electronically, magnetically, optically, chemically, mechanically, or otherwise, and can include electronic, magnetic, optical, electromagnetic, infrared, or semiconductor components.

Aspects can take the form of a computer program product embodied in one or more tangible non-transitory computer readable medium(s) having computer readable program code embodied thereon. Such medium(s) can be manufactured as is conventional for such articles, e.g., by pressing a CD-ROM. The program embodied in the medium(s) includes computer program instructions that can direct data processing system 1110 to perform a particular series of operational steps when loaded, thereby implementing functions or acts specified herein.

In an example, data storage system 1140 includes code memory 1141, e.g., a random-access memory, and disk 1142, e.g., a tangible computer-readable rotational storage device such as a hard drive. Computer program instructions are read into code memory 1141 from disk 1142, or a wireless, wired, optical fiber, or other connection. Data processing system 1110 then executes one or more sequences of the computer program instructions loaded into code memory 1141, as a result performing process steps described herein. In this way, data processing system 1110 carries out a computer implemented process. For example, blocks of the flowchart illustrations or block diagrams herein, and combinations of those, can be implemented by computer program instructions.

Computer program code can be written in any combination of one or more programming languages, e.g., Java, Smalltalk, C++, C, or an appropriate assembly language. Program code to carry out methods described herein can execute entirely on a single data processing system 1110 or on multiple communicatively-connected data processing systems 1110. For example, code can execute wholly or partly on a user's computer and wholly or partly on a remote computer, e.g., a server. The remote computer can be connected to the user's computer through network 1150. The user's computer or the remote computer can be non-portable computers, such as conventional desktop personal computers (PCs), or can be portable computers such as tablets, cellular telephones, smartphones, or laptops.

The peripheral system **1120** can include one or more devices configured to provide digital content records or other information to the data processing system **1110.** For example, the peripheral system **1120** can include digital still cameras, digital video cameras, cellular phones, or other data processors. The data processing system **1110,** upon receipt of digital content records from a device in the peripheral system **1120,** can store such digital content records in the data storage system **1140.** Peripheral system 1120 can include or be communicatively connected to an insulin pump, a continuous glucose monitor having one or more glucose sensor(s), an episodic glucose monitor (e.g., a finger-stick test-strip-based blood glucose sensor), or one or more physiological sensor(s) such as those described above (e.g., a heart rate monitor). The peripheral system 1120 can include a detector, e.g., a barcode or RFID reader, to retrieve insulin type information stored in or encoded on the surface of a vial or other container of insulin. In embodiments implemented at least partly in devices worn by the user or integrated into the user's clothing or personal effects, peripheral system 1120 can include an accelerometer that provides information to permit data processing system 1110 to infer the user's level of activity from the frequency, duration, amplitude, or other properties of motions made by the user.

The user interface system **1130** can include a mouse, a keyboard, another computer (connected, e.g., via a network or a null-modem cable), or any device or combination of devices from which data is input to the data processing system **1110.** In this regard, although the peripheral system **1120** is shown separately from the user interface system **1130,** the peripheral system **1120** can be included as part of the user interface system **1130.** User interface system 1130 can include controls operable by a user to indicate to data processing system 1110 the user's activity level or the type of insulin being administered by the user.

The user interface system **1130** also can include a display device, a processor-accessible memory, or any device or combination of devices to which data is output by the data processing system **1110.** In this regard, if the user interface system **1130** includes a processor-accessible memory, such memory can be part of the data storage system **1140** even though the user interface system **1130** and the data storage system **1140** are shown separately in FIG. 3.

In various aspects, data processing system 1110 includes communication interface 1115 that is coupled via network link 1116 to network 1150. For example, communication interface 1115 can be an integrated services digital network (ISDN) card or a modem to provide a data communication connection to a corresponding type of telephone line. As another example, communication interface 1115 can be a network card to provide a data communication connection to a compatible local-area network (LAN), e.g., an Ethernet LAN, or wide-area network (WAN). Wireless links, e.g., WiFi or GSM, can also be used. Communication interface 1115 sends and receives electrical, electromagnetic or optical signals that carry digital data streams representing various types of information across network link 1116 to network 1150. Network link 1116 can be connected to network 1150 via a switch, gateway, hub, router, or other networking device.

Network link 1116 can provide data communication through one or more networks to other data devices. For example, network link 1116 can provide a connection through a local network to a host computer or to data equipment operated by an Internet Service Provider (ISP).

Data processing system 1110 can send messages and receive data, including program code, through network 1150, network link 1116 and communication interface 1115. For example, a server can store requested code for an application program (e.g., a JAVA applet) on a tangible non-volatile computer-readable storage medium to which it is connected. The server can retrieve the code from the medium and transmit it through the Internet, thence a local ISP, thence a local network, thence communication interface 1115. The received code can be executed by data processing system 1110 as it is received, or stored in data storage system 1140 for later execution.

While the invention has been described in terms of particular variations and illustrative figures, those of ordinary skill in the art will recognize that the invention is not limited to the variations or figures described. For example, the closed-loop controller need not be an MPC controller but can be, with appropriate modifications by those skilled in the art, a PID controller, a PID controller with internal model control (IMC), a model-algorithmic-control (MAC) that are discussed by Percival et al., in "Closed-Loop Control and Advisory Mode Evaluation of an Artificial Pancreatic β Cell: Use of Proportional-Integral-Derivative Equivalent Model-Based Controllers" Journal of Diabetes Science and Technology, Vol. 2, Issue 4, July 2008. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above.

## Claims

1. A method to control an infusion pump responsive to a controller that receives data from at least one glucose sensor, the method comprising automatically performing the following steps using the controller:

   computing insulin time-action model parameters, a, b and c, by applying a machine-learning process to the received data of the physiological variables; the parameter a being a function of the last dose of insulin, carbohydrate and fat content of a meal, a patient's nominal Insulin Sensitivity Factor;
   the parameter b being a function of the carbohydrate and fat content of meals;
   the parameter c being a constant that depends on the type of insulin; receiving from the glucose sensor respective glucose level measurements for each time interval of a series of discrete time intervals;
   calculating an insulin delivery amount for a selected one of the time intervals by:

   predicting a trend of the glucose level from estimates of a metabolic state of the subject using a plurality of the respective glucose level measurements and glucose infusion rate, GIR, calculated based on an insulin time-action model of the form:

   $$GIR(t) = at^{-1}e^{-b[\log(t)-c]^2};$$

   and
   using a model predictive controller, determining the insulin delivery amount to provide a desired future glucose level in response to the predicted trend; and

   commanding the infusion pump to deliver the calculated insulin delivery amount.

2. Apparatus for the delivery of insulin, the apparatus comprising:

a) a glucose monitor adapted to continually measure respective glucose levels of a patient at discrete time intervals and provide respective glucose measurement data indicating each measured glucose level;
b) an insulin infusion pump configured to deliver insulin in response to a delivery control signal;
c) an interface adapted to receive data of one or more physiological variables; and
d) a controller adapted to, for each of a plurality of the discrete time intervals:

i) receive data of physiological variables including last dose of insulin, carbohydrate and fat content of a meal, a patient's nominal Insulin Sensitivity Factor and an insulin-type dependent constant, but not including glucose level of the patient;
ii) computing insulin time-action model parameters, a, b and c, using the received data of the physiological variables by applying a machine-learning process to the received data of the physiological variables;
iii) predict a trend of the glucose level from estimates of a metabolic state of the subject using a plurality of the respective glucose level measurements and glucose infusion rate, GIR, calculated based on an insulin time-action model of the form:

$$GIR(t) = at^{-1}e^{-b[\log(t)-c]^2};$$

iv) using a model predictive controller, determine the insulin delivery amount to provide a desired future glucose level in response to the predicted trend; and
v) provide to the insulin infusion pump a delivery control signal corresponding to the determined insulin delivery amount, whereby a corresponding amount of insulin is delivered.

**Patentansprüche**

1. Verfahren zur Steuerung einer Infusionspumpe, die auf einen Controller anspricht, der Daten von mindestens einem Glucosesensor empfängt, wobei das Verfahren die automatische Durchführung der folgenden Schritte unter Anwendung des Controllers umfasst:

Berechnen von Zeitaktionsmodellparametern für Insulin a, b und c durch Anwenden eines Maschinenlernprozesses auf die empfangenen Daten der physiologischen Variablen;
wobei der Parameter a eine Funktion der letzten Insulindosis, des Kohlehydrat- und Fettgehalts einer Mahlzeit und des nominellen Insulinsensivitätsfaktors eines Patienten ist;
der Parameter b eine Funktion des Kohlehydrat- und Fettgehalts von Mahlzeiten ist;
der Parameter c eine Konstante ist, die vom Insulintyp abhängt;
Empfangen vom Glucosesensor jeweilige Glucosespiegelmessungen für jedes Zeitintervall einer Reihe von diskreten Zeitintervallen;
Berechnen einer Insulingabemenge für ein gewähltes Zeitintervall durch:

Vorhersagen eines Trends des Glucosespiegels aus Schätzungen eines Stoffwechselzustands des Patienten unter Anwendung von mehreren der jeweiligen Glucosespiegelmessungen und Glucoseinfusionsrate GIR, die auf der Basis eines Zeitaktionsmodells für Insulin mit der folgenden Formel beruht:

$$GIR(t) = at^{-1}e^{-b[\log(t)-c]^2};$$

und
unter Anwendung eines modellprädiktiven Controllers Bestimmen der Insulingabemenge, um einen gewünschten zukünftigen Glucosespiegel in Reaktion auf den vorhergesagten Trend zu liefern; und

Befehlen der Infusionspumpe, die berechnete Insulingabemenge abzugeben.

2. Vorrichtung für die Insulingabe, umfassend:

a) einen Glucosemonitor, der angepasst ist, um kontinuierlich die jeweiligen Glucosespiegel eines Patienten in diskreten Zeitintervallen, zu messen und die jeweiligen Glucosemessdaten, die jeden gemessenen Glucosespiegel angeben, bereitzustellen;

b) eine Insulininfusionspumpe, die konfiguriert ist, um Insulin in Reaktion auf ein Abgabesteuersignal abzugeben;

c) eine Schnittstelle, die angepasst ist, um Daten von einer oder mehreren physiologischen Variablen zu empfangen; und

d) einen Controller, der angepasst ist, um für jeden von mehreren diskreten Zeitintervallen:

i) Daten von physiologischen Variablen zu empfangen, welche die letzte Insulindosis, Kohlehydrat- und Fettgehalt einer Mahlzeit, den nominellen Insulinsensitivitätsfaktor eines Patienten und eine vom Insulintyp abhängige Konstante, doch nicht den Glucosespiegel des Patienten enthalten;

ii) Zeitaktionsmodellparameter für Insulin a, b und c unter Anwendung der empfangenen Daten der physiologischen Variablen durch Anwenden eines Maschinenlernprozesses auf die empfangenen Daten der physiologischen Variablen zu berechnen;

iii) einen Trend des Glucosespiegels aus Schätzungen eines Stoffwechselzustands des Patienten unter Anwendung von mehreren jeweiligen Glucosespiegelmessungen und der Glucoseinfusionsrate GIR vorherzusagen, die auf der Basis eines Zeitaktionsmodells für Insulin mit folgender Formel berechnet wird:

$$GIR(t) = at^{-1}e^{-b[\log(t)-c]^2};$$

iv) unter Anwendung eines modellprädiktiven Controllers die Insulingabemenge zu bestimmen, um einen gewünschten zukünftigen Glucosespiegel in Reaktion auf den vorhergesagten Trend bereitzustellen; und

v) an die Insulininfusionspumpe ein Abgabesteuersignal zu liefern, das der bestimmten Insulingabemenge entspricht, wodurch eine entsprechende Insulinmenge abgegeben wird.

## Revendications

1. Procédé pour commander une pompe à perfusion en réponse à un organe de commande qui reçoit des données en provenance d'au moins un capteur de glucose, le procédé consistant à réaliser automatiquement les étapes suivantes à l'aide de l'organe de commande :

calculer des paramètres de modèle temps-action d'insuline, a, b et c, en appliquant un processus d'apprentissage machine aux données reçues des variables physiologiques ;

le paramètre a étant une fonction de la dernière dose d'insuline, de la teneur en glucide et graisse d'un repas, du facteur de sensibilité à l'insuline nominale d'un patient ;

le paramètre b étant une fonction de la teneur en glucide et graisse des repas ;

le paramètre c étant une constante qui dépend du type d'insuline ;

recevoir en provenance du capteur de glucose des mesures respectives du taux de glucose pour chaque intervalle de temps d'une série d'intervalles de temps discrets ;

calculer une quantité d'administration d'insuline pour un intervalle de temps sélectionné des intervalles de temps par :

la prédiction d'une tendance du taux de glucose à partir d'estimations d'un état métabolique du sujet à l'aide d'une pluralité des mesures respectives du taux de glucose et de la vitesse de perfusion du glucose, GIR, calculée sur la base d'un modèle temps-action de l'insuline sous la forme suivante :

$$GIR(t) = at^{-1}e^{-b[\log(t)-c]^2};$$

et

à l'aide d'un organe de commande prédictif de modèle, la détermination de la quantité d'administration d'insuline pour fournir un taux de glucose futur souhaité en réponse à la tendance prédite ; et

commander à la pompe à perfusion d'administrer la quantité d'administration d'insuline calculée.

2. Appareil pour l'administration d'insuline, l'appareil comprenant :

a) un moniteur de glucose conçu pour mesurer en continu des taux de glucose respectifs d'un patient à des intervalles de temps discrets et fournir des données de mesure du glucose respectives indiquant chaque taux de glucose mesuré ;
b) une pompe à perfusion d'insuline conçue pour administrer de l'insuline en réponse à un signal de commande d'administration ;
c) une interface conçue pour recevoir des données d'une ou de plusieurs variables physiologiques ; et
d) un organe de commande conçu, pour chaque intervalle de la pluralité d'intervalles de temps discrets, pour :

i) recevoir des données de variables physiologiques comprenant la dernière dose d'insuline, la teneur en glucide et graisse d'un repas, le facteur de sensibilité à l'insuline nominale d'un patient et une constante dépendant du type d'insuline, mais ne comprenant pas le taux de glucose du patient ;
ii) calculer des paramètres de modèle temps-action d'insuline, a, b et c, à l'aide des données reçues des variables physiologiques en appliquant un processus d'apprentissage machine aux données reçues des variables physiologiques ;
iii) prédire une tendance du taux de glucose à partir d'estimations d'un état métabolique du sujet à l'aide d'une pluralité de mesures respectives du taux de glucose et de la vitesse de perfusion du glucose, GIR, calculée sur la base d'un modèle temps-action de l'insuline sous la forme suivante :

$$GIR(t) = at^{-1}e^{-b[\log(t)-c]^2} \quad ;$$

iv) à l'aide d'un organe de commande prédictif de modèle, déterminer la quantité d'administration d'insuline pour fournir un taux de glucose futur souhaité en réponse à la tendance prédite ; et
v) fournir à la pompe à perfusion d'insuline un signal de commande d'administration correspondant à la quantité d'administration d'insuline déterminée, moyennant quoi une quantité correspondante d'insuline est administrée.

**FIG. 1**

**FIG. 2**

EP 2 973 095 B1

1150 — NETWORK

1116 —

1115 —

1130 —

1110 — DATA PROCESSING SYSTEM

USER INTERFACE SYSTEM

1120 — PERIPHERAL SYSTEM

1140 —

CODE MEMORY

DISK

1141

1142

DATA STORAGE SYSTEM

## FIG. 3

**FIG. 4** *PRIOR ART*

Plasma Insulin Levels

Aspart, lispro, glulisine

Regular

NPH

Detemir

Glargine

510

Hours

0  2  4  6  8  10  12  14  16  18  20  22  24

*FIG. 5* <u>*PRIOR ART*</u>

**FIG. 6**

EP 2 973 095 B1

$X_2$ ACTIVITY
$X_3$ ISF
$X_4$ CARB
$X_5$ FAT
INSULIN TYPE

$X_1$ HEART RATE

A

B

C

RTC

GIR(T)

**FIG. 7**

BEGIN

START INSULIN PUMP AND SENSORS. SET INSULIN TYPE.

BEGIN PERIODIC COLLECTION OF SENSOR DATA

AT EACH CYCLE (TIME): READ ALL SENSORS AND FEED DATA TO CORRESPONDING PARAMETERS

UPDATE BAYES NETWORK PARAMETERS WITH NEW SENSOR INFORMATION

RUN MODEL EQUATION TO PRODUCE NEW INSULIN TIME-ACTION CURVE

DETERMINE INSULIN AMOUNT

DELIVER DETERMINED INSULIN AMOUNT

NO      SHUTDOWN PUMP?      YES      END

**FIG. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7060059 B
- US 8062249 B
- US 20110313680 A
- US 20110257627 A
- WO 2012051344 A
- US 20110208156 A
- WO 2003080157 A
- WO 2010135646 A
- WO 03080157 A
- US 6883148 B, Teig
- US 6857112 B, Teig
- US 20030088320 A1
- US 20030232314 A1
- US 20040131107 A1
- US 20040250166 A1
- US 20050105682 A1
- US 20050123893 A1
- US 20070091085 A1
- US 20080052312 A1
- US 20080065471 A1
- US 20080208581 A1
- US 20090004638 A1
- US 20090144101 A1
- US 20090144123 A1
- US 20090161741 A1
- US 20090279737 A1
- US 20100076913 A1
- US 20100135584 A1
- US 20100161611 A1
- US 20100262568 A1
- US 20100280804 A1
- US 20110087673 A1
- US 20110153419 A1
- US 20110206246 A1
- US 20110231704 A1
- US 20110256607 A1

- US 20110302031 A1
- US 20110314039 A1
- US 20120059253 A1
- US 20120143789 A1
- US 20120155717 A1
- US 20120202212 A1
- US 20120271556 A1
- US 6687887 B1
- US 6735748 B1
- US 6832069 B2
- US 6857112 B1
- US 6883148 B1
- US 6892366 B1
- US 6907591 B1
- US 7051293 B1
- US 7085690 B2
- US 7099435 B2
- US 7103524 B1
- US 7213174 B2
- US 7457581 B2
- US 7684651 B2
- US 7755619 B2
- US 7756682 B1
- US 7860347 B2
- US 7865336 B1
- US 7877234 B1
- US 7974570 B2
- US 8090665 B2
- US 8170905 B2
- US 8204838 B2
- US 8229165 B2
- US 8234274 B2
- US 8280705 B2
- US 8301482 B2
- US 7276029 B

### Non-patent literature cited in the description

- **PERCIVAL et al.** Closed-Loop Control and Advisory Mode Evaluation of an Artificial Pancreatic β Cell: Use of Proportional-Integral-Derivative Equivalent Model-Based Controllers. *Journal of Diabetes Science and Technology,* July 2008, vol. 2 (4
- **PAOLA SORU et al.** MPC Based Artificial Pancreas; Strategies for Individualization and Meal Compensation. *Annual Reviews in Control,* 2012, vol. 36, 118-128

- **COBELLI et al.** Artificial Pancreas: Past, Present, Future. *Diabetes,* November 2011, vol. 60
- **MAGNI et al.** Run-to-Run Tuning of Model Predictive Control for Type 1 Diabetes Subjects: In Silico Trial. *Journal of Diabetes Science and Technology,* September 2009, vol. 3 (5

- **LEE et al.** A Closed-Loop Artificial Pancreas Using Model Predictive Control and a Sliding Meal Size Estimator. *Journal of Diabetes Science and Technology,* September 2009, vol. 3 (5
- **LEE et al.** A Closed-Loop Artificial Pancreas based on MPC: Human Friendly Identification and Automatic Meal Disturbance Rejection. *Proceedings of the 17th World Congress, The International Federation of Automatic Control,* 06 July 2008
- **MAGNI et al.** Model Predictive Control of Type 1 Diabetes: An in Silico Trial. *Journal of Diabetes Science and Technology,* November 2007, vol. 1 (6
- **WANG et al.** Automatic Bolus and Adaptive Basal Algorithm for the Artificial Pancreatic $\beta$-Cell. *Diabetes Technology and Therapeutics,* 2010, vol. 12 (11
- **PERCIVAL et al.** Closed-Loop Control of an Artificial Pancreatic $\beta$-Cell Using MultiParametric Model Predictive Control. *Diabetes Research,* 2008
- **COBELLI et al.** Diabetes: Models, Signals, and Control. *IEEE Reviews in Biomedical Engineering,* 2009, vol. 2
- **PERCIVAL et al.** Closed-Loop Control and Advisory Mode Evaluation of an Artificial Pancreatic [beta] Cell: Use of Proportional-Integral-Derivative Equivalent Model-Based Controllers. *Journal of Diabetes Science and Technology,* July 2008, vol. 2 (4
- **DALLA MAN et al.** Physical Activity into the Meal Glucose-Insulin Model of Type 1 Diabetes: In Silico Studies Author Affiliations. *Journal of Diabetes Science and Technology,* January 2009, vol. 3 (1
- **WILINSKA et al.** Simulation Environment to Evaluate Closed-Loop Insulin Delivery Systems in Type 1 Diabetes Author Affiliations. *Journal of Diabetes Science and Technology,* January 2010, vol. 4 (1
- **BERNARDO ; SMITH.** Bayesian Theory. John Wiley & Sons Ltd, 2000
- **CARL EDWARD RASMUSSEN.** Bayesian Learning in Feed Forward Neural Networks. Department of Computer Science at the University of Toronto
- **N. METROPOLIS ; A. W. ROSENBLUTH ; M. N. ROSENBLUTH ; A. H. TELLER ; E. TELLER.** Equation of state calculations by fast computing machines. *Journal of Chemical Physics,* vol. 21, 1087-1092
- **S. DUANE ; A. D. KENNEDY ; B. J. PENDLETON ; D. ROWETH.** Hybrid Monte Carlo. *Physics Letters,* vol. 195, 216-222
- Technical Report CRG-TR-91-1 of the Connectionist Research Group. **RADFORD M. NEAL.** Bayesian Training of Backpropagation Networks by the Hybrid Monte Carlo Method. Department of Computer Science at the University of Toronto, 1992
- Bayesian Learning for Neural Networks. **RADFORD M. NEAL'S.** PhD thesis. Department of Computer Science at the University of Toronto, 1995
- **CARL EDWARD RASMUSSEN.** A Practical Monte Carlo Implementation of Bayesian Learning. Department of Computer Science at the University of Toronto
- **KOVATCHEV et al.** In Silico Preclinical Trials: A Proof of Concept in Closed-Loop Control of Type 1 Diabetes. *J Diabetes Sci Technol.,* January 2009, vol. 3 (1), 44-55
- **BENYAMIN GROSMAN, PH.D. ; EYAL DASSAU, PH.D. ; HOWARD C. ZISSER, M.D. ; LOIS JOVANOVIČ, M.D. ; FRANCIS J. DOYLE III, PH.D.** Zone Model Predictive Control: A Strategy to Minimize Hyper and Hypoglycemic Events. *Journal of Diabetes Science and Technology,* July 2010, vol. 4 (4
- **MACIEJOWSKI JM.** PREDICTIVE CONTROL, WITH CONSTRAINTS. Prentice-Hall, Pearson Education Limited, 2002
- **RACHEL GILLIS et al.** Glucose Estimation and Prediction through Meal Responses Using Ambulatory Subject Data for Advisory Mode Model Predictive Control. *Journal of Diabetes Science and Technology,* November 2007, vol. 1 (6
- **YOUQING WANG et al.** Closed-Loop Control of Artificial Pancreatic $\beta$-Cell in Type 1 Diabetes Mellitus Using Model Predictive Iterative Learning Control. *IEEE Transactions on Biomedical Engineering,* February 2010, vol. 57 (2